# EUROPÄISCHE PATENTSCHRIFT

(11) **EP 1 216 692 B1**
(45) Veröffentlichungstag und Bekanntmachung des Hinweises auf die Patenterteilung: **21.02.2007**
(21) Anmeldenummer: 01128695.2
(22) Anmeldetag: 01.12.2001
(51) Int. Cl.: A61K 8/49, A61Q 19/08

(54) **Verwendung von alpha-CEHC in kosmetischen Zubereitungen**
Use of alpha-CEHC in cosmetic preparations
Utilisation de l'alpha-CEHC dans des compositions cosmétiques

(30) Priorität: 22.12.2000 DE 10064818
(43) Veröffentlichungstag der Anmeldung: 26.06.2002
(73) Patentinhaber: BASF Aktiengesellschaft, 67056 Ludwigshafen (DE)
(72) Erfinder: Krämer, Klaus, Dr., 76829 Landau (DE); Jaedicke, Hagen, Dr., 67069 Ludwigshafen (DE); Jentzsch, Axel, Dr., 68167 Mannheim (DE)

(56) Entgegenhaltungen:
- EP-A- 0 238 302
- WO-A-00/57889
- WO-A-93/17659
- DE-A- 19 903 716
- US-A- 5 315 017
- PATENT ABSTRACTS OF JAPAN Bd. 0062, Nr. 50 (C-139), 9. Dezember 1982 (1982-12-09) & JP 57 145871 A (EISAI KK), 9. September 1982 (1982-09-09) -& DATABASE CAPLUS [Online] ACS; 1982, XP002261666 gefunden im STN Database accession no. 98:71933/DN
- PLACER, Z. ET AL.: DIE NAHRUNG, Bd. 12, Nr. 4, 1968, Seiten 491-492, XP0001091318
- DATABASE WPI Section Ch, Week 200116 Derwent Publications Ltd., London, GB; Class B02, AN 2001-151009 XP002261667 & JP 2000 290276 A (CCI KK) 17. Oktober 2000 (2000-10-17)
- JIANG ET AL: "Gamma-tocopherol and its major metabolite, in contrast to alpha-tocopherol, inhibit cyclooxygenase activity in macrophages and epithelial cells" PROCEEDINGS OF THE NATIONAL ACADEMY OF SCIENCES OF USA, NATIONAL ACADEMY OF SCIENCE. WASHINGTON, US, Bd. 97, Nr. 21, 10. Oktober 2000 (2000-10-10), Seiten 11494-11499, XP002190542 ISSN: 0027-8424

## Beschreibung

Die vorliegende Erfindung betrifft die Verwendung von Chroman-Derivaten in kosmetischen Zubereitungen. Insbesondere betrifft die vorliegende Erfindung kosmetische Zubereitungen zur Prophylaxe gegen Alterungsprozesse in der Haut.

Die menschliche Haut unterliegt gewissen Alterungsprozessen, die teilweise auf intrinsische Prozesse (chronoaging) und teilweise auf exogene Faktoren (environmental, z.B. photoaging) zurückzuführen sind. Zusätzlich können vorübergehende oder auch andauernde Veränderungen des Hautbildes auftreten, wie Akne, fettige oder trockene Haut, Keratosen, Rosaceae, lichtempfindliche, entzündliche, erythematöse, allergische oder autoimmunreaktive Reaktionen wie Dermatosen und Photodermatosen.

Zu den exogenen Faktoren zählen insbesondere das Sonnenlicht oder künstliche Strahlungsquellen mit vergleichbarem Spektrum sowie Verbindungen, die durch die Strahlung entsteher können, wie undefinierte reaktive Photoprodukte, die auch radikalisch oder ionisch sein können. Zu diesen Faktoren zählen auch Zigarettenrauch und die darin enthaltenen reaktiven Verbindungen wie Ozon, freie Radikale, beispielsweise das Hydroxylradikal, Singulettsauerstoff und andere reaktive Sauerstoff- oder Stickstoffverbindungen, die die natürliche Physiologie oder Morphologie der Haut stören.

Durch den Einfluß dieser Faktoren kann es unter anderem zu direkten Schäden an der DNA der Hautzellen kommen sowie an den Kollagen-, Elastin- oder Glycosaminoglycanmolekülen der extrazellulären Matrix, die für die Festigkeit der Haut verantwortlich sind. Darüberhinaus kann es zu einer Beeinflussung der Signaltransduktionsketten kommen, an deren Ende die Aktivierung matrixabbauender Enzyme steht. Wichtige Vertreter dieser Enzyme sind die Matrixmetalloproteinasen (MMPs, z.B. Kollagenasen, Gelatinasen, Stromelysine), deren Aktivität zusätzlich durch TIMPs (tissue inhibitor of matrix metalloproteinases) reguliert wird.

Die Folgen der o.g. Alterungsprozesse sind Verdünnung der Haut, schwächere Verzahnung von Epidermis und Dermis, Reduktion der Zellzahl sowie der versorgenden Blutgefäße. Dabei kommt es zur Ausbildung von feinen Linien und Falten, die Haut wird ledrig und es können Pigmentstörungen auftreten.

Die gleichen Faktoren wirken auch auf Haare, wo es ebenfalls zu einer Schädigung kommen kann. Die Haare werden spröde, weniger elastisch und glanzlos. Die Oberflächenstruktur der Haare ist geschädigt.

Kosmetische oder dermatologische Pflegeprodukte mit Eigenschaften, die den beschriebenen oder vergleichbaren Prozessen entgegenwirken oder deren schädliche Folgen mindern oder rückgängig machen sollen, zeichnen sich häufig durch folgende spezifische Eigenschaften aus - radikalfangend, antioxidativ, entzündungshemmend oder feuchthaltend wirksam. Sie verhindern oder reduzieren u.a. die Aktivität der matrixabbauenden Enzyme oder regulieren die Neusynthese von Kollagen, Elastin oder Proteoglycanen.

Die Verwendung von Antioxidantien oder Radikalfängern in kosmetischen Zubereitungen ist an sich hinlänglich bekannt. So ist der Einsatz des antioxidativen Vitamin E in Sonnenschutzformulierungen bereits Stand der Technik (z. B. De Polo, KF "A Short Textbook of Cosmetology" 1998). Dennoch bleibt auch hier die erzielte Wirkung hinter der erhofften weit zurück.

Aufgrund des immer größer werdenden Bedarfs an kosmetischen Wirkstoffen zur vorbeugenden Behandlung von menschlicher Haut und menschlicher Haare gegen Alterungsprozesse und schädigenden Umwelteinflüßen war es Aufgabe der vorliegenden Erfindung, neue Wirkstoffe für die kosmetische Anwendung bereitzustellen, die die bereits eingangs genannten kosmetischen Wirkungen zeigen, ausreichend oxidations- und photostabil sowie gut formulierbar sein sollen. Die damit hergestellten kosmetischen Zubereitungen sollen ferner ein möglichst niedriges Irritationspotential für die Haut aufweisen, sie sollen die Wasserbindung in der Haut positiv beeinflussen, die Elastizität der Haut erhöhen und somit eine Glättung der Haut bewirken. Darüberhinaus sollen sie beim Auftragen auf die Haut ein angenehmes Hautgefühl erzeugen.

Diese Aufgade wurde gelöst durch die Verwendung von 2,5,7,8-Tetramethyl-2-(β-Carboxyethyl)-6-hydroxychroman [α-CEHC] der Formel Ia 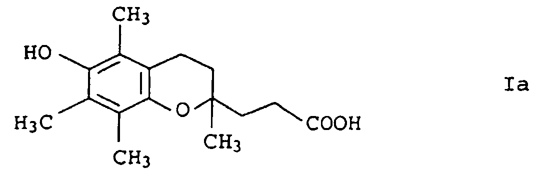 α -CEHC ist als natürliches Abbauprodukt von α-Tocopherol bekannt. Es findet sich sowohl im Urin als auch im Blutserum des Menschen (Schultz et al., Am. J. Clin. Nutr., 62(suppl.), 1527S-1534S, 1995; W. Stahl et al., Analytical Biochemistry, 275, 254-259, 1999).

Die Anwendung von α-CEHC in der Kosmetik war bislang nicht bekannt - weder zur Vorbeugung noch zur Behandlung von Hautschäden.

Es war daher überraschend, dass mit α-CEHC sowohl ein vorbeugender Effekt gegen Hautschäden, insbesondere ein vorbeugender Effekt gegen Alterungsprozesse der menschlichen Haut, als auch eine kosmetische Wirkung gegen bereits aufgetretene Hautschäden erzielt werden kann.

Die erfindungsgemäße Verwendung von der Verbindung der Formel Ia in kosmetischen Zubereitungen bietet u.a. einen Schutz vor Schäden, die durch UV-Strahlung oder durch reaktive Verbindungen hervorgerufene Prozesse direkt oder indirekt verursacht werden, wie z. B.
- der Hautalterung,
- dem Verlust der Hautfeuchtigkeit,
- dem Verlust der Hautelastizität,
- der Bildung von Falten oder Runzeln oder
- von Pigmentstörungen oder Altersflecken.

Weiterhin betrifft die vorliegende Erfindung die Verwendung der o.g. Zubereitungen zur kosmetischen Vorbeugung unerwünschter Veränderungen des Hautbildes, wie z.B.
- Akne oder fettige Haut,
- Keratosen,
- Rosaceae,
- lichtempfindliche, entzündliche, erythematöse, allergische oder autoimmunreaktive Reaktionen.

Ferner bieten die erfindungsgemäß Verbindung der Formel Ia in haarkosmetischen Zubereitungen einen Schutz gegen vorzeitige Alterungsprozesse der menschlichen Haare und können somit als Wirkstoffe bei der kosmetischen Behandlung von sprödem, glanzlosem und unelastischem Haar eingesetzt werden.

Zur Anwendung werden die erfindungsgemäßen kosmetischen Zubereitungen in der für Kosmetika üblichen Weise auf die Haut und/oder die Haare in einer ausreichend wirksamen Menge aufgebracht.

Erfindungsgemäße Zubereitungen können z. B. eine Lösung, eine wasserfreie Zubereitung, eine Emulsion oder Mikroemulsion vom Typ Wasser-in-Öl oder vom Typ Öl-in-Wasser, eine multiple Emulsion, beispielsweise vom Typ Wasser-in-Öl-in-Wasser, ein Gel, einen festen Stift, eine Salbe, ein Aerosol oder auch ein wässriges System bzw. eine Tensidzubereitung zur Reinigung von Haut und/oder Haaren, darstellen.

Es ist auch vorteilhaft, die Verbindungen der Formel I in verkapselter Form darzureichen, z. B. als Celluloseverkapselung, in Gelatine, Wachsmatrices, mit Cyclodextrinen oder liposomal verkapselt.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der oben genannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen. Sofern Vitamin A bzw. Vitamin-A-Derivate bzw. Carotine bzw. deren Derivate das oder die Antixoidantien darstellen, ist es vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstereat, Isopropyloleat, n-Butylstereat, N-Hexyllaurat, N-Decyloleat, Isooctylstearat, Isononylstearat, Isononylisononanoat, 2-Ethylhexylpalmitat, 2-Ethylhexyllaurat, 2-Hexyldecylstereat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylether.

Besonders vorteilhaft sind Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisonnanoat, aus Cyclomethicon und 2-Ethylhexylisostearat.

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Es ist weiterhin vorteilhaft, zusätzliche öllösliche organische UV-A-Filter und/oder UV-B-Filter in der Lipidphase und/oder wasserlösliche organische UV-A-Filter und/oder UV-B-Filter in der wäßrigen Phase einzusetzen, wobei die Gesamtmenge der Filtersubstanzen z.B. 0,1 Gew.-% bis 30 Gew.-%, vorzugsweise 0,5 bis 15 Gew.-%, insbesondere 1 bis 10 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Lichtschutzmittel, die alleine oder als Gemisch zusammen mit der Verbindung der Formel Ia verwendet werden können sind z.B.

**Tabelle 1:**

| Nr. | Stoff | CAS-Nr. (=Säure) |
|---|---|---|
| 1 | 4-Aminobenzoesäure | 150-13-0 |
| 2 | 3-(4'-Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxy-ethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfobenzyliden)-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | Menthyl-o-aminobenzoate oder: 5-Methyl-2-(1-methylethyl)-2-aminobenzoat | 134-09-8 |
| 20 | Glyceryl p-aminobenzoat oder: 4-Aminobenzoesäure-1-glyceryl-ester | 136-44-7 |
| 21 | 2,2'-Dihydroxy-4-methoxybenzophenon (Dioxy-benzone) | 131-53-3 |
| 22 | 2-Hydroxy-4-methoxy-4-methylbenzophenon (Mexenon) | 1641-17-4 |
| 23 | Triethanolamin Salicylat | 2174-16-5 |
| 24 | Dimethoxyphenylglyoxalsäure oder: 3,4-dimethoxy-phenyl-glyoxal-saures Natrium | 4732-70-1 |
| 25 | 3-(4'Sulfobenzyliden)-bornan-2-on und seine Salze | 56039-58-8 |
| 26 | 2,2,4,4-Tetrahydroxybenzophenon | 131-55-5 |
| 27 | 2,2-Methylen-bis-[6(2H-benzotriazol-2-yl)-4-(1,1,3,3,-tetramethylbutyl)phenol] | 103597-45-1 |
| 28 | 2,2-(1,4-Phenylen)-bis-1H-benzimidazol-4,6-disulfonsäure, Na-Salz | 180898-37-7 |
| 29 | 2,4-bis-[4-(2-Ethylhexyloxy)-2-hydroxy]phenyl-6-(4-methoxyphenyl)-(1,3,5)-triazin | 187393-00-6 |
| 30 | 3-(4-Methylbenzyliden)-campher | 36861-47-9 |
| 31 | 4-Bis(polyethoxy)paraaminobenzoesäurepolyethoxyethylester | 113010-52-9 |
| 32 | 2,4-Dihydroxybenzophenon | 131-56-6 |
| 33 | 2,2'-Dihydroxy-4,4'-dimethoxybenzophenon-5,5'-dinatriumsulfonat | 3121-60-6 |

Weitere kombinierbare Lichtschutzmittel sind u.a. folgende Verbindungen:

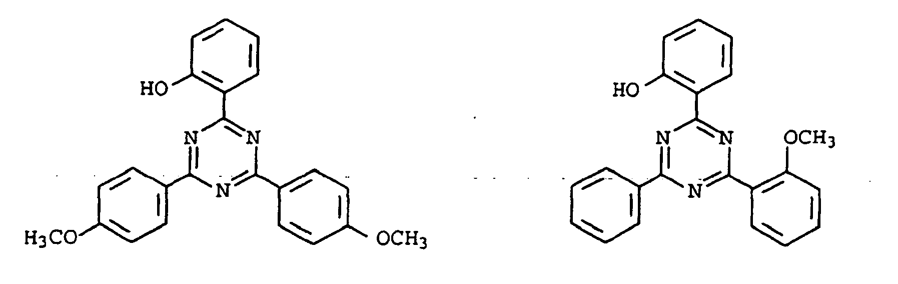

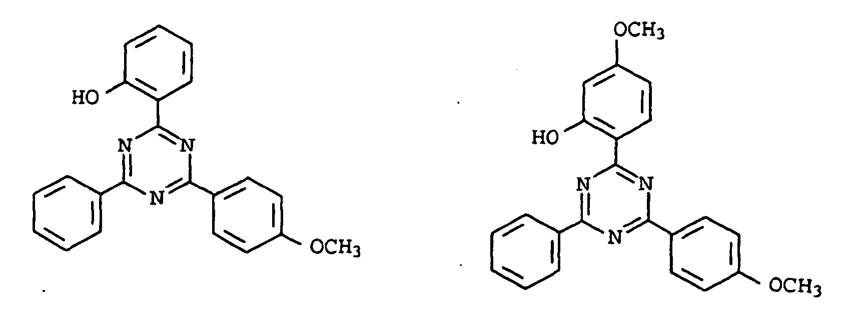

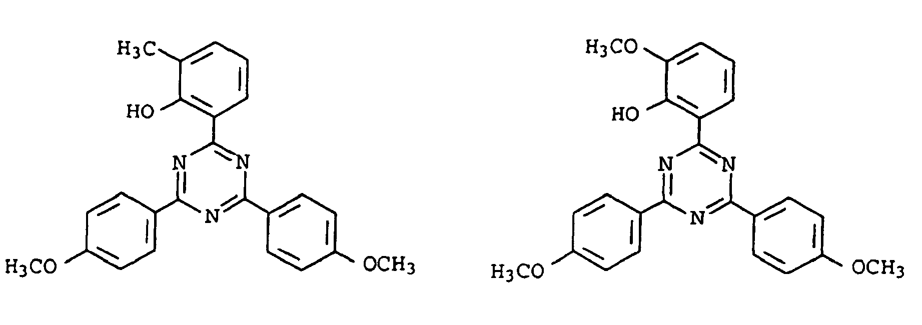

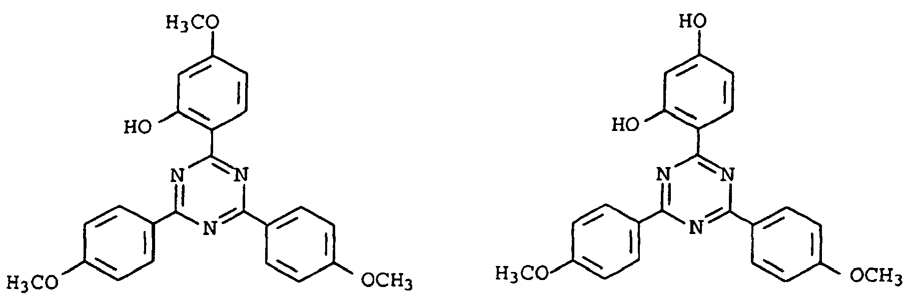

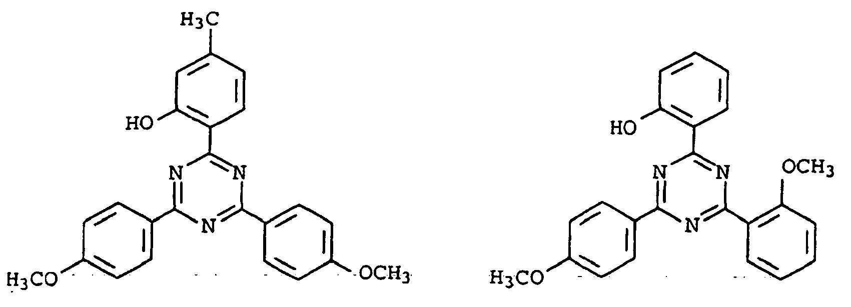

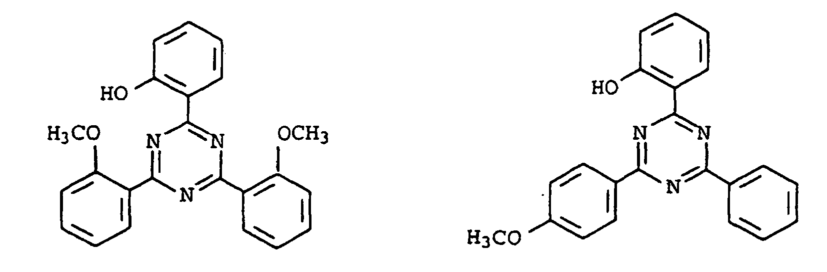

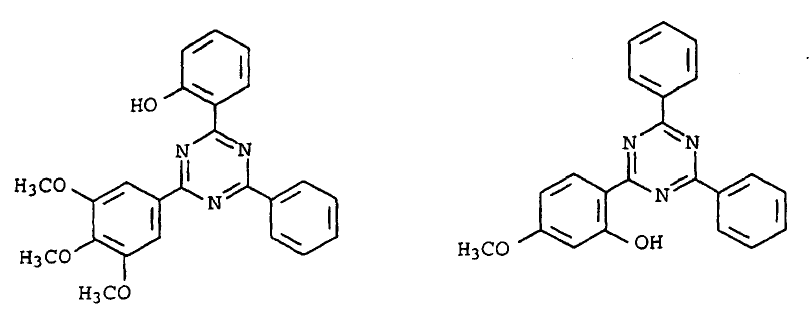

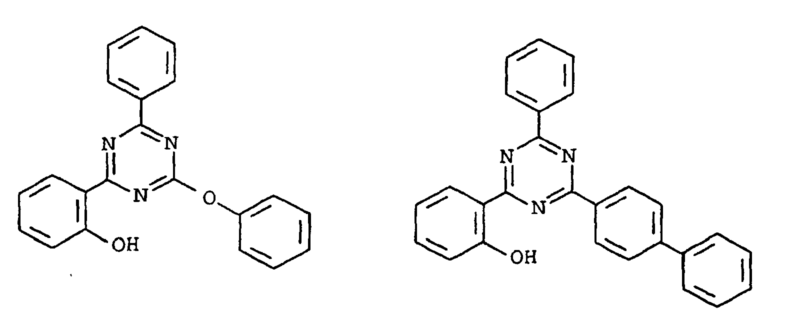

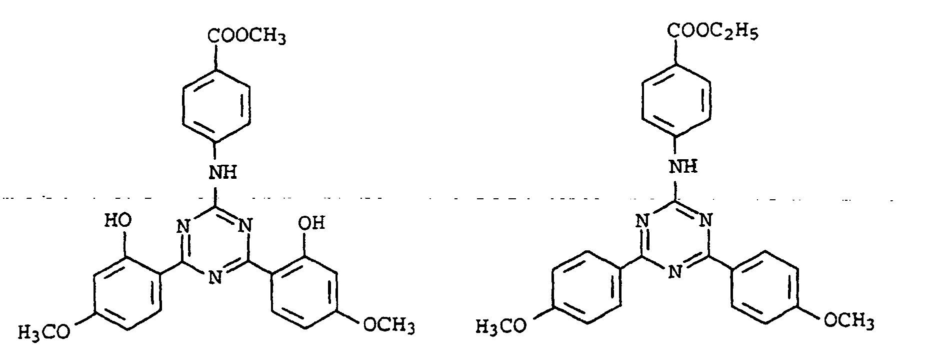

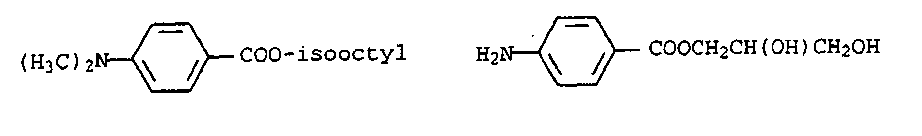

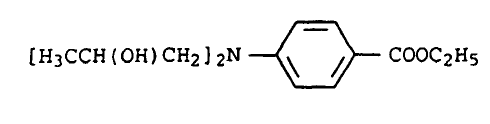

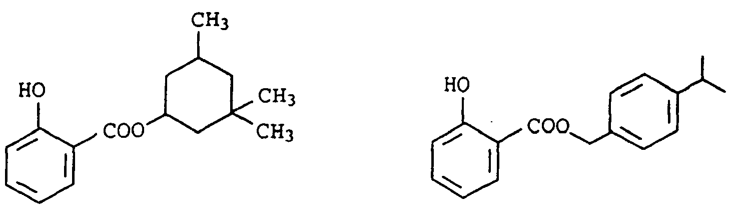

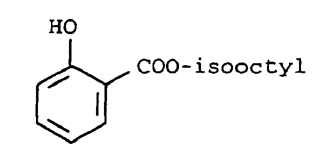

Die Liste der genannten UV-Filter, die in Kombination mit den erfindungsgemäß verwendeten Chroman-Derivaten eingesetzt werden können, soll selbstverständlich nicht limitierend sein.

Kosmetische und dermatologische Zubereitungen mit einem wirksamen Gehalt an mindestens einem Chroman-Derivat der Formel Ia können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Bei kosmetischen und dermatologischen Zubereitungen zum Schutze der Haare gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier-und Behandlungslotion, einen Haarlack oder um Dauerwellmittel.

Die kosmetischen und dermatologischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie mindestens ein Chroman-Derivat der Formel Ia in wirksamer Konzentration. Die Menge der verwendeten Polymeren liegt z.B. zwischen 0,1 und 10 Gew.-%, bevorzugt zwischen 0,1 und 3 Gew.-%.

Kosmetische Zubereitungen zur Behandlung und Pflege der Haare, die mindestens ein Chroman-Derivat der Formel Ia enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an mindestens einem Chroman-Derivat der Formel Ia und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge an Chroman-Derivat der Formel Ia in einem für die Haare bestimmten Mittel 0,01 bis 30 Gew.-%, bevorzugt 0,05 bis 5 Gew.-%, insbesondere 0,1 bis 2 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine Fettsäurealkanolamide
- Polyglycolether-Derivate.

Kosmetische Zubereitungen, die kosmetische Reinigungszubereitungen für die Haut darstellen, können in flüssiger oder fester Form vorliegen. Sie enthalten neben mindestens einem Chroman-Derivat der Formel I vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an mindestens einem Chroman-Derivat der Formel Ia vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die vorliegende Erfindung umfaßt auch ein kosmetisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an mindestens einem Chroman-Derivat der Formel I enthält, in ausreichender Menge auf die Haut oder Haare aufbringt.

Ebenso umfaßt die vorliegende Erfindung auch ein Verfahren zum Schutze kosmetischer oder dermatologischer Zubereitungen gegen Oxidation oder Photooxidation, wobei diese Zubereitungen z.B. Zubereitungen zur Behandlung und Pflege der Haare darstellen, insbesondere Haarfärbemittel, Haarlacke, Shampoonierungsmittel, Farbshampoonierungsmittel, ferner Schminkprodukte wie z.B. Nagellacke, Lippenstifte, Teintgrundlagen, Wasch- und Duschzubereitungen, Cremes zur Behandlung oder Pflege der Haut oder um sämtliche anderen kosmetischen Zubereitungen handelt, deren Bestandteile Stabilitätsprobleme aufgrund von Oxidation bzw. Photooxidation bei der Lagerung mit sich bringen können, dadurch gekennzeichnet, daß die kosmetischen Zubereitungen einen wirksamen Gehalt an mindestens einem Chroman-Derivat der Formel Ia aufweisen.

In den nachfolgenden Beispielen werden Zusammensetzungen von kosmetischen Formulierungen, die die Verbindung der Formel Ia enthalten, näher erläutert.

**Beispiel 1**

| Soft Skin Fluid | |
|---|---|
| | Massengehalt (Gew.-%) |
| Ceteareth-6 and Stearyl Alcohol | 2,50 |
| Ceteareth-25 | 2,50 |
| Hydrogenated Coco-Glycerides | 1,50 |
| PEG-40 Dodecyl Glycol Copolymer | 3,00 |
| Dimethicone | 3,00 |
| Phenethyl Dimethicone | 2,00 |
| Cyclomethicone | 1,00 |
| Cetearyl Octanoate | 5,00 |
| Avocado Oil | 1,00 |
| Sweet Almond Oil | 2,00 |
| Wheat Germ Oil | 0,80 |
| α-CEHC | 0,20 |
| Panthenol USP | 1,00 |
| Phytantriol | 0,20 |
| Tocopheryl Acetat | 0,30 |
| Propylene Glycol | 5,00 |
| Fragrance | q.s. |
| Preservative | q.s. |
| Aqua | ad 100 |

**Beispiel 2**

| Hand Protection Cream | |
|---|---|
| | Massengehalt (Gew.-%) |
| Cetearyl Alcohol | 1,00 |
| Glyceryl Stearate | 1,50 |
| Stearyl Alcohol | 1,50 |
| Cetyl Palmitat | 2,00 |
| Tocopheryl Acetate | 0,50 |
| Dimethicone | 8,00 |
| Ceteareth-6 and Stearyl Alcohol | 3,00 |
| Octyl Methoxycinnamate | 5,00 |
| Propylenglycol | 8,00 |
| Panthenol | 1,00 |
| Evening Primrose Oil | 3,00 |
| PEG-7 Hydrogenated Castor Oil | 6,00 |
| Glyceryl Oleate | 1,00 |
| Phenethyl Dimethicone | 3,00 |
| Beeswax | 1,50 |
| Locust Bean Gum | 0,80 |
| Silkpowder | 0,80 |
| Preservative | q.s. |
| Fragrance | q.s. |
| Borax | 0,10 |
| α-CEHC | 0,20 |
| Aqua | ad 100 |

**Beispiel 3**

| Sun Care Lotion | |
|---|---|
| | Massengehalt (Gew.-%) |
| PEG-7 Hydrogenated Castor Oil | 6,00 |
| PEG-40 Hydrogenated Castor Oil | 0,50 |
| Isopropyl Palmitate | 7,00 |
| PEG-45/Dodecyl Glycol Coplymer | 2,00 |
| Jojoba Oil | 3,00 |
| Magnesium Stearate | 0,60 |
| Octyl Methoxycinnamate | 8,00 |
| C₁₂₋₁₅ Alkyl Benzoate | 5,00 |
| Titanium Dioxide | 4,00 |
| Propylene Glycol | 5,00 |
| EDTA | 0,20 |
| Preservative | q.s. |
| Sodium Ascorbyl Phosphate | 1,00 |
| Tocopheryl Acetate | 0,50 |
| α-CEHC | 0,20 |
| Fragrance | q.s. |
| Aqua | ad 100 |

**Beispiel 4**

| Multiple Emulsion | |
|---|---|
| | Massengehalt (Gew.-%) |
| Mineral Oil | 7,50 |
| Cetearyl Octanoate | 2,50 |
| Aluminium Stearate | 0,25 |
| Magnesium Stearate | 0,25 |
| Microdristalline Wax H | 0,50 |
| Cetearyl Alcohol | 1,00 |
| Lanolin Alcohol | 1,50 |
| Mineral Alcohol and Lanolin Alcohol | 1,50 |
| PEG-7 Hydrogenated Castor Oil | 0,75 |
| PEG-45/Dodecyl Glycol Copolymer | 2,00 |
| Ceteareth-6 and Stearyl Alcohol | 2,00 |
| Ceteareth-25 | 2,00 |
| Trilauret-4 Phosphate | 1,00 |
| Hydroxyethylcellulose | 0,20 |
| Propylenglycol | 7,50 |
| Magnesium Sulfate | 0,25 |
| α-CEHC | 0,10 |
| Water | ad 100 |

**Beispiel 5**

| Microemulsion | |
|---|---|
| | Massengehalt (Gew.-%) |
| Ceteareth-25 | 13,00 |
| PEG-7 Glyceryl Cocoate | 20,00 |
| Octyl Dodecanol | 5,00 |
| Preservative | q.s. |
| α-CEHC | 0,20 |
| Aqua | ad 100 |

**Beispiel 6**

| Liposomengel | |
|---|---|
| | Massengehalt (Gew.-%) |
| PEG-40 Hydrogenated Castor Oil | 1,00 |
| Bisabolol rac. | 0,10 |
| Propylene Glycol | 8,00 |
| Panthenol | 0,50 |
| Tocopheryl Acetate and Polysorbate 80 | |
| and Caprylic/Capric Triglyceride | |
| and Lecithin | 3,00 |
| Preservative | q.s. |
| Parfum | q.s. |
| Carbomer | 0,50 |
| α-CEHC | 0,20 |
| Triethanolamine | 0,70 |
| Aqua | ad 100 |

**Beispiel 7**

| Blunted Oil Gel | |
|---|---|
| | Massengehalt (Gew.-%) |
| Silica | 5,00 |
| Dimethicone | 10,00 |
| Cetearyl Octanoate | 40,00 |
| Caprylic / Capric Triglyceride | 8,00 |
| Phenethyl Dimethicone | 2,00 |
| Mineral Oil | 28,50 |
| Sweet Almond Oil | 5,00 |
| Phytantriol | 0,30 |
| α-CEHC | 0,10 |
| Tocopherol | 0,50 |
| Fragrance | 1,00 |

**Beispiel 8**

| Oil Gel | |
|---|---|
| | Massengehalt (Gew.-%) |
| Silica | 5,00 |
| Dimethicone | 10,00 |
| Cetearyl Octanoate | 30,00 |
| Caprylic / Capric Triglyceride | 10,00 |
| Isopropylmyristate | 5,00 |
| Phenethyl Dimethicone | 5,00 |
| Mineral Oil | 28,20 |
| Jojoba Oil | 5,00 |
| Phytantriol | 0,30 |
| α-CEHC | 0,30 |
| Tocopherol | 0,50 |
| Fragrance | 1,00 |

**Beispiel 9**

| Sun Care Lip Protection Stick | |
|---|---|
| | Massengehalt (Gew.-%) |
| Beeswax | 12,00 |
| Hydrogenated Coco Glycerides | 5,00 |
| Ricinus Oil | 40,00 |
| Isopropylpalmitate | 10,00 |
| Mineral Oil | 10,00 |
| Candelilla Wax | 8,00 |
| Phenethyl Dimethicone | 5,00 |
| α-CEHC | 0,20 |
| Petrolatum | 5,00 |
| Benzophenone-3 | 5,00 |

**Beispiel 10**

| Cooling Body Splash | |
|---|---|
| | Massengehalt (Gew.-%) |
| PEG-40 Hydrogenated Castor Oil | 2,00 |
| Menthyl Lactate | 0,20 |
| Alcohol | 5,00 |
| PEG-7 Glyceryl Cocoate | 2,00 |
| Witch Hazel | 5,00 |
| Allantoin | 0,10 |
| Bisabolol rac. | 0,20 |
| Propylenglycol | 5,00 |
| Panthenol USP | 0,50 |
| Lactic Acid (80%ig) | 0,20 |
| α-CEHC | 0,20 |
| Fragrance | q.s. |
| Aqua | ad 100 |

**Beispiel 11**

| Make-up | |
|---|---|
| | Massengehalt (Gew.-%) |
| Ceteareth-6 and Stearyl Alcohol | 9,00 |
| Dimethicone | 5,00 |
| Cetearyl Octanoate | 8,00 |
| Macadamia Nut Oil | 5,00 |
| Propylenglycol | 5,00 |
| Sicovit White E 171 | 8,00 |
| Sicomet Brown 70 13E 3717 | 2,00 |
| α-CEHC | 0,20 |
| Fragrance | q.s. |
| Benzophenone-3 | 5,00 |
| Aqua | ad 100 |

**Beispiel 12**

| Fluid Make-up | |
|---|---|
| | Massengehalt (Gew.-%) |
| Ceteareth-6 and Stearyl Alcohol | 7,00 |
| Ceteareth-25 | 5,00 |
| Dimethicone | 5,00 |
| Cetearyl Octanoate | 8,00 |
| Macadamia Nut Oil | 5,00 |
| Propylenglycol | 5,00 |
| Sicovit Weiss E 171 | 8,00 |
| Sicomet Braun 70 13E 3717 | 1,00 |
| α-CEHC | 0,10 |
| Fragrance | q.s. |
| Benzophenone-3 | 5,00 |
| Aqua | ad 100 |

**Beispiel 13**

| Sun Care Oil | |
|---|---|
| | Massengehalt (Gew.-%) |
| Cetearyl Octanoate | 40,00 |
| Caprylic/Capric Triglyceride | 28,70 |
| Evening Primrose Oil | 3,00 |
| Macadamia Nut Oil | 5,00 |
| Isopropylpalmitate | 5,00 |
| Dimethicone | 3,00 |
| Octyl Methoxycinnamate | 8,00 |
| Octocrylene | 5,00 |
| Benzophenone-3 | 2,00 |
| Phytantriol | 0,10 |
| α-CEHC | 0,10 |
| Tocopheryl Acetate | 0,20 |
| Fragrance | q.s. |

**Beispiel 14**

| Facial Scrub Cleanser | |
|---|---|
| | Massengehalt (Gew.-%) |
| Cocoamidopropyl Betain | 5,00 |
| Potassium Coco-Hydrolyzed | |
| Animal Protein | 8,00 |
| PEG-40 Hydrogenated Castor Oil | 2,00 |
| Polyquaternium-44 | 7,70 |
| Bisabolol rac. | 0,20 |
| Panthenol | 1,00 |
| Fragrance | 0,50 |
| Hydroxyethyl Cellulose | 2,00 |
| α-CEHC | 0,50 |
| Propylenglycol | 5,00 |
| Jojoba Wax | 3,00 |
| Water | ad 100 |

**Beispiel 15**

| Conditioner | |
|---|---|
| | Massengehalt (Gew.-%) |
| Ceteareth-6 and Stearyl Alcohol | 2,00 |
| Ceteareth-25 | 1,00 |
| Cetearyl Octanoate | 6,00 |
| Ceteareth-3 | 2,00 |
| Cetearyl Alcohol | 6,00 |
| Phytantriol | 1,00 |
| Propylene Glycol | 5,00 |
| Polyquaternium-11 | 5,00 |
| Panthenol | 1,00 |
| Retinyl Acetate | 0,50 |
| Fragrance | q.s. |
| α-CEHC | 0,50 |
| Preservative | q.s. |
| Aqua | ad 100 |

**Beispiel 16**

| Hair Wax | |
|---|---|
| | Massengehalt (Gew.-%) |
| Polyethylenglycol-6 | 30,00 |
| Polyethylenglycol-75 | 45,00 |
| Paraffinum Liquidum | 0,50 |
| PEG-40 Hydrogenated Castor Oil | 1,00 |
| Glycerin | 15,00 |
| Benzophenone-3 | 2,00 |
| Phytantriol | 0,10 |
| α-CEHC | 0,30 |
| Fragrance | q.s. |
| Aqua | ad 100 |

**Beispiel 17**

| Anti-Dandruff Hair Tonic | |
|---|---|
| | Massengehalt (Gew.-%) |
| Alcohol | 45,00 |
| Aloe Vera (10fach Konz.) | 1,00 |
| Panthenol | 1,00 |
| Tocopheryl Acetat | 0,50 |
| PEG-40 Hydrogenated Castor Oil | 0,50 |
| Allantoin | 0,10 |
| Hydrolyzed Animal Protein | 1,50 |
| 1-(4-Chlorphenoxy)-1-(1H-imidazolyl)-3,3-dimethyl-2-butanon | 0,30 |
| Fragrance | 0,10 |
| α-CEHC | 0,40 |
| Aqua | ad 100 |

**Beispiel 18**

| Foot Deo Spray | |
|---|---|
| | Massengehalt (Gew.-%) |
| PEG-40 Hydrogenated Castor Oil | 0,80 |
| Alcohol | 20,00 |
| Farnesol | 0,12 |
| Menthyl Lactat | 0,08 |
| 1,2-Propylenglycol | 3,20 |
| Benzophenone-4 | 1,20 |
| PEG-7 Glyceryl Cocoate | 0,80 |
| Fragrance | q.s. |
| α-CEHC | 0,40 |
| Butan | 60,00 |
| Aqua | ad 100 |

**Beispiel 19**

| Hair Spray | |
|---|---|
| | Massengehalt (Gew.-%) |
| Aminomethyl Propanol | 0,40 |
| Dimethicone Copolyol | 0,03 |
| Alcohol | 43,67 |
| Pentane | 14,20 |
| Acrylates/Acrylamide Copolymer | 3,40 |
| Fragrance | q.s. |
| α-CEHC | 0,30 |
| Butane | 2,40 |
| Iso-Butane | ad 100 |

## Patentansprüche

1. Verwendung eines Chroman-Derivaten mit der Formel la 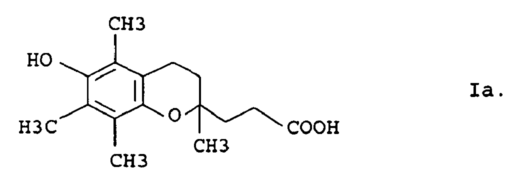 in kosmetischen oder dermatologischen Zubereitungen.

2. Verwendung nach Anspruch 1 zur Prophylaxe gegen Alterungsprozesse der menschlichen Haut.

3. Verwendung nach Anspruch 2 zur Prophylaxe gegen trockene Haut, Faltenbildung und/oder Pigmentstörungen.

4. Verwendung nach Anspruch 1 zur Prophylaxe gegen Alterungsprozesse menschlicher Haare.

5. Verwendung nach einem der Ansprüche 1 bis 4, **dadurch gekennzeichnet, daß** die Verbindung der Formel la in einem wirksamen Gehalt in kosmetischen Zubereitungen vorliegt.

6. Verwendung nach Anspruch 5, **dadurch gekennzeichnet, daß** die Verbindung der Formel la in Konzentrationen von 0,01 bis 30 Gew.-%, bezogen auf die Gesamtmenge der kosmetischen Zubereitung, vorliegt.

## Claims

1. The use of a chroman derivative having the formula Ia, 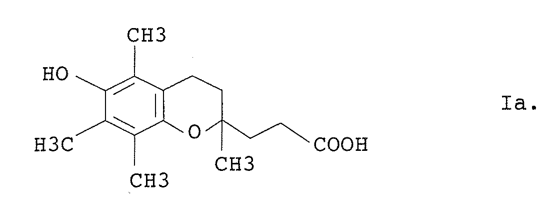 in cosmetic or dermatological preparations.

2. The use according to claim 1 for prophylaxis against aging processes of the human skin.

3. The use according to claim 2 for prophylaxis against dry skin, wrinkle formation and/or pigment disorders.

4. The use according to claim 1 for prophylaxis against aging processes of human hair.

5. The use according to any of claims 1 to 4, wherein the compound of the formula Ia is present in an effective content in cosmetic preparations.

6. The use according to claim 5, wherein the compound of the formula Ia is present in concentrations of from 0.01 to 30% by weight, based on the total amount of the cosmetic preparation.

## Revendications

1. Utilisation d'un dérivé de chromane répondant à la formule Ia : 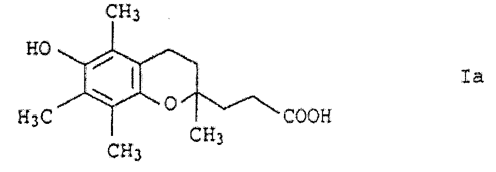 dans des compositions cosmétiques ou dermatologiques.

2. Utilisation suivant la revendication 1, pour la prophylaxie vis-à-vis de processus de vieillissement de la peau humaine.

3. Utilisation suivant la revendication 2, pour la prophylaxie vis-à-vis de peau sèche, de formation de rides et/ou de troubles pigmentaires.

4. Utilisation suivant la revendication 1, pour la prophylaxie vis-à-vis de processus de vieillissement des cheveux humains.

5. Utilisation suivant l'une des revendications 1 à 4, **caractérisée en ce que** le composé de la formule Ia se présente en une teneur efficace dans des compositions cosmétiques.

6. Utilisation suivant la revendication 5, **caractérisée en ce que** le composé de la formule Ia se présente en des concentrations de 0,01 à 30 % en poids, par rapport à la quantité totale de la composition cosmétique.
